# EUROPÄISCHE PATENTANMELDUNG

(11) **EP 0 902 043 A2**
(43) Veröffentlichungstag der Anmeldung: **17.03.1999**
(21) Anmeldenummer: 98116886.7
(22) Anmeldetag: 07.09.1998
(51) Int. Cl.: C08G 79/00, C07D 345/00

(54) **Polyselenophene, Verfahren zu deren Herstellung, und deren Verwendung**

(30) Priorität: 10.09.1997 DE 19739775
(71) Anmelder: BASF AKTIENGESELLSCHAFT, 67056 Ludwigshafen (DE)
(72) Erfinder: Möhwald, Helmut, Dr., 76855 Annweiler (DE); Belov, Vladimir, Dr., 67069 Ludwigshafen (DE); Schrof, Wolfgang, Dr., 67271 Neuleiningen (DE)
(74) Vertreter: Isenbruck, Günter, Dr.

(57) **Zusammenfassung**

Neue Polyselenophene enthaltend eine oder mehrere Struktureinheiten der allgemeinen Formeln (I) oder (II) oder (I) und (II) 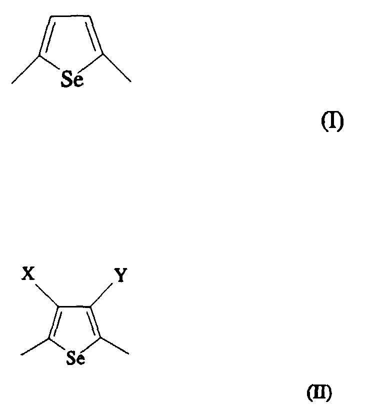 Verfahren zu ihrer Herstellung, Monomere zu deren Herstellung, ihre Verwendung in verschiedenen technischen Gebieten, sowie ein diese enthaltendes elektrisch leitfähiges Material.

## Beschreibung

Die vorliegende Erfindung betrifft neue Polyselenophene. Ferner betrifft sie Verfahren und Monomere zur Herstellung von Polyselenophenen, deren Verwendung in verschiedenen technischen Gebieten, sowie ein elektrisch leitfähiges Material, das mindestens ein erfindungsgemäßes Polyselenophen enthält.

Als konjugierte Polymere mit hoher elektrischer Leitfähigkeit und nichtlinearen optischen Eigenschaften sind u.a. Polythiophene bekannt. Diese können als Materialien für Halbleiter verwendet werden (s.u.a. EP-B-0 332 704 und P.N. Prasad, D.J. Williams "Introduction to NLO effects in molecules and polymers", John Wiley and Sons, Inc. (1991)). Ferner besitzen die Polythiophene Elektrochromie- und antistatische Eigenschaften (s. N. Arsalani und K.E. Geckeler "Conducting isopolymers: peparation, properties, and applications", J. Prakt. Chem. 337 (1995), S. 1-11.

Weitere Einzelheiten besonders gut verwendbarer Polythiophene sind der PCT/EP97/01140 und der darin zitierten Literatur zu entnehmen.

Die bislang bekannten elektrisch leitfähigen Materialien waren nicht immer in der Lage den wachsenden Anforderungen an diese Materialien, wie z.B. vergleichsweise hohe elektrische Leitfähigkeit bei gleichzeitig guten mechanischen Eigenschaften zu erfüllen. Diese Überlegungen zeigen beispielhaft die Notwendigkeit, neue, bislang unbekannte dotierte und undotierte Poly- und Oligomere zu entwickeln.

Somit lag die Aufgabe der vorliegenden Erfindung in der Bereitstellung neuer Polyselenophene, die in vorteilhafter Weise wie oben skizziert in verschiedenen technischen Bereichen angewendet werden können.

Diese Aufgabe wird durch die im folgenden näher beschriebenen erfindungsgemäßen Polyselenophene erfüllt.

Die vorliegende Erfindung betrifft somit ein Polyselenophen enthaltend eine oder mehrere Struktureinheiten der allgemeinen Formeln (I) oder (II) oder (I) und (II) 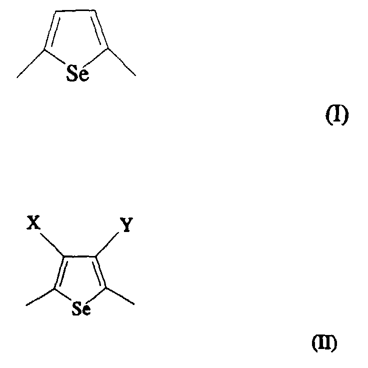 wobei
X und Y unabhängig voneinander gleich oder verschieden sein können und Wasserstoff, mit der Maßgabe, daß nicht sowohl X als auch Y Wasserstoff ist; eine lineare oder verzweigtkettige C₁- bis C₂₂-Alkygruppe; eine lineare oder verzweigtkettige C₁- bis C₂₂-Alkoxygruppe; eine lineare oder verzweigtkettige C₁- bis C₂₂-Alkyloxyalkylgruppe; eine lineare oder verzweigtkettige C₁- bis C₂₂-Acylgruppe; eine lineare oder verzweigtkettige C₁- bis C₂₂-Thioacylgruppe; eine lineare oder verzweigtkettige C₁- bis C₂₂-Acyloxygruppe; eine lineare oder verzweigtkettige C₁- bis C₂₂-Thioacyloxygruppe; eine C₅- bis C₈-Cycloalkylgruppe, eine C₆- bis C₁₈-Arylgruppe oder eine C₅- bis C₈-heterocyclische Gruppe, die jeweils wiederum mit einer oder mehreren linearen oder verzweigtkettigen C₁- bis C₂₂-Alkylgruppe(n), einer oder mehreren linearen oder verzweigtkettigen C₁- bis C₂₂-Alkoxygruppe(n), einer oder mehreren linearen oder verzweigtkettigen C₁- bis C₂₂-Alkyloxyalkylgruppe(n), einer oder mehreren linearen oder verzweigtkettigen C₁- bis C₂₂-Acylgruppe(n) oder einer oder mehreren linearen oder verzweigtkettigen C₁- bis C₂₂-Thioacylgruppe(n) substituiert sein können; NO₂; oder NHR¹, wobei R¹ gleich oder verschieden sein kann und jeweils Wasserstoff, eine lineare oder verzweigtkettige C₁- bis C₂₂-Alkylgruppe, eine lineare oder verzweigtkettige C₁- bis C₂₂-Alkoxygruppe, eine lineare oder verzweigtkettige C₁- bis C₂₂-Alkyloxyalkylgruppe, eine lineare oder verzweigtkettige C₁- bis C₂₂-Acylgruppe oder eine lineare oder verzweigtkettige C₁- bis C₂₂-Thioacylgruppe ist, bedeuten, oder X und Y zusammen mit den Atomen, an die sie gebunden sind, ein Kohlenstoff enthaltendes Ringsystem bilden, das neben Kohlenstoff Stickstoff (N)-, Sauerstoff (O)-, Schwefel (S)- oder Phosphor (P)-Heteroatome oder Mischungen zweier oder mehrerer dieser Heteroatome aufweist,
   wobei dieses Ringsystem wiederum an dem (den) Kohlenstoffatom(en), dem (den) Stickstoffatom(en) oder dem (den) Phosphoratom(en) jeweils mit einer Gruppe Z substituiert sein kann, in der jedes Z unabhängig voneinander eine Gruppe wie oben für X und Y definiert bedeutet, oder
zwei benachbarte Gruppen Z zusammen einen durch eine der folgenden allgemeinen Formeln (III) bis (VI) dargestellten Rest bilden 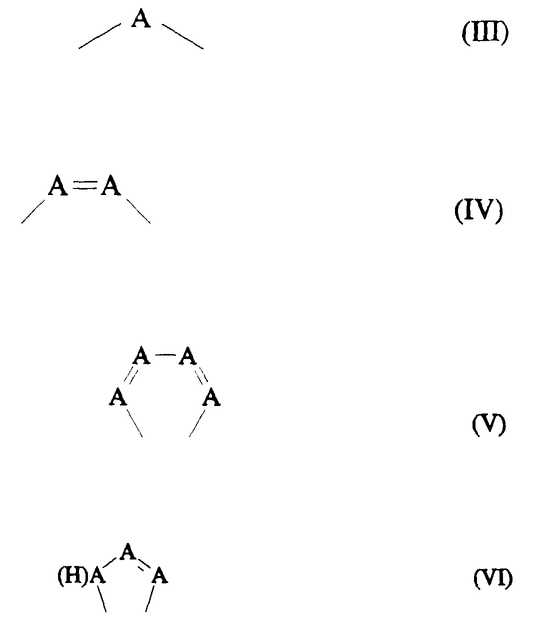 in denen A Kohlenstoff (C), Stickstoff (N), Phosphor (P) oder Mischungen zweier oder mehrerer dieser Atome bedeutet,
   wobei, sofern A Kohlenstoff ist, jedes dieser A entweder ein Wasserstoffatom trägt oder wiederum wie oben für X und Y definiert, substituiert sein kann.

In einer weiteren Ausführungsform enthält das erfindungsgemäße Polyselenophen eine oder mehrere Thiophen-Struktureinheiten der allgemeinen Formeln (XII) oder (XIII) oder (XII) und (XIII) 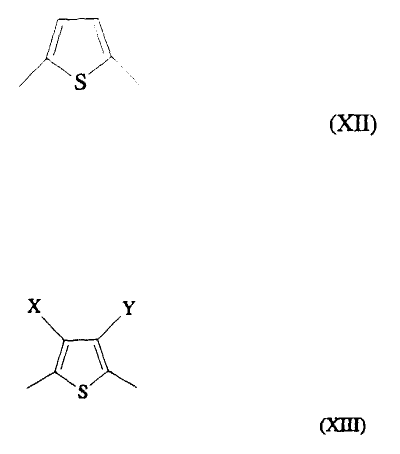 wobei
X und Y unabhängig voneinander gleich oder verschieden sein können und wie oben für die Polyselenophen-Einheiten definiert sind.

Bevorzugte Ausführungsformen der obigen Thiophen-Einheiten sowie die Herstellung und Eigenschaften der entsprechenden diesen Einheiten zugrundeliegenden Verbindungen sind der eingangs erwähnten Anmeldung PCT/EP97/01140 zu entnehmen, die diesbezüglich vollumfänglich in den Kontext der vorliegenden Anmeldung einbezogen wird.

Der Begriff "Polyselenophen", wie er im Rahmen der vorliegenden Anmeldung verwendet wird, umfaßt alle Poly- und Oligomere, die eine oder mehrere der Einheiten der allgemeinen Formeln (I) und/oder (II) enthalten. Dies umfaßt sowohl das Vorhandensein einer oder mehrerer gleicher Einheiten (I) und/oder (II) als auch das Vorhandensein von mehreren verschiedenen Einheiten (II), die dann jeweils wiederum mindestens einmal in dem erfinungsgemäßen Polyselenophen vorhanden sein können. Vorzugsweise enthalten die erfindungsgemäßen Polyselenophene eine oder mehrere Einheiten der allgemeinen Formeln (I) und (II). Ferner werden auch alle Poly- und Oligomere umfaßt, die eine oder mehrere der Einheiten der allgemeinen Formeln (I) und/oder (II) zusammen mit einer oder mehreren Thiophen-Einheiten der allgemeinen Formeln (XII) und/oder (XIII) enthalten.

Darüberhinaus werden auch Kombinationen der Einheiten der allgemeinen Formeln (I) und/oder (II) mit anderen konjugierten organischen Struktureinheiten, wie z.B. Phenylen-, Biphenylen-, Terphenylen-, Naphthalen- Anthracen-, Furan-Einheiten, wobei diese wiederum mono- oder polysubstituiert sein können, wie oben für X und Y definiert, umfaßt.

Die in den erfindungsgemäßen Polyselenophenen vorhandene Struktureinheit (I) leitet sich von Selenophen ab.

In der Struktureinheit (II), in der X und Y, sofern sie nicht zusammen mit den Atomen, an die sie gebunden sind, ein Kohlenstoff enthaltendes Ringsystem bilden, jeweils die eingangs erwähnten Gruppen darstellen können, ist es bevorzugt, daß X und Y gleich sind. Sofern X und Y jeweils die Gruppe NHR¹ darstellen, sind die beiden Reste R¹ vorzugsweise ebenfalls identisch. Unter den oben aufgelisteten Alkyl-, Alkoxy-, Alkyloxyalkyl-, Acyl- und Thioacylgruppen mit 1 bis 22 C-Atomen sind solche mit 1 bis 20 bevorzugt, solche mit 6 bis 20 Kohlenstoffatomen weiter bevorzugt und solche mit 10 bis 16 Kohlenstoffatomen besonders bevorzugt.

Als Substituenten X und Y besonders bevorzugt sind die oben definierten Acylgruppen, Thioacylgruppen und NHR¹.

Im Rahmen der vorliegenden Erfindung bezeichnet der Ausdruck "Thioacylgruppe" eine Gruppe der allgemeinen Formel ―C(S)―R, wobei R Alkyl ist. Der Ausdruck "heterocyclische Gruppe", wie eingangs erwähnt, bezeichnet alicyclische gesätigte, alicyclische ungesättigte und aromatische heterocyclische Gruppen.

Ferner können X und Y zusammen mit den Atomen, an die sie gebunden sind, ein Kohlenstoff enthaltendes Ringsystem bilden, das neben Kohlenstoff Stickstoff (N)-, Sauerstoff (O)-, Schwefel (S)- oder Phosphor (P)-Heteroatome oder Mischungen zweier oder mehrerer dieser Heteroatome aufweist. Vorzugsweise bilden dabei X und Y einen zweiwertigen Rest, der zwei bis acht Atome, weiter bevorzugt drei bis sechs Atome, aufweist, und, zusammen mit den beiden Atomen, an die er gebunden ist, ein Ringsystem mit vier bis zehn bzw. fünf bis acht Atomen bildet. Die Anzahl der in diesem Ringsystem möglicherweise vorhandenen, oben definierten Heteroatome beträgt vorzugsweise bis zu drei, weiter bevorzugt bis zu zwei. Unter den oben definierten Heteroatomen ist Stickstoff (N) bevorzugt. Vorzugsweise handelt es sich bei dem oben beschriebenen Ringsystem um ein mindestens eine, weiter bevorzugt zwei oder mehr Doppelbindungen aufweisendes System. In besonders bevorzugten Ausführungsformen der vorliegenden Erfindung befinden sich die Doppelbindungen des obigen Ringsystems in Konjugation mit den Doppelbindungen des Selenophenfragments, an das das Ringsystem gebunden ist, und ggf. den Doppelbindungen weiterer an das oben beschriebene Ringsystem gebundener Reste der Formeln (III) bis (VI), wie oben definiert.

Dieses Ringsystem kann wiederum an den Kohlenstoff-, Stickstoff- oder Phosphoratomen jeweils mit einer Gruppe Z substituiert sein, wobei jedes Z unabhängig voneinander eine Gruppe wie oben für X und Y definiert, bedeutet, und auch hier die für X und Y als bevorzugt angegebenen Substituenten wiederum als bevorzugt anzusehen sind.

Zwei benachbarte Gruppen Z können darüber hinaus zusammen einen Rest bilden, der ausgewählt wird unter den eingangs erwähnten Resten der allgemeinen Formeln (III) bis (VI), wobei die Platzhalter "A" Kohlenstoff (C), Stickstoff (N), Phosphor (P) oder Mischungen zweier oder mehrerer dieser Atome bedeuten, wobei, sofern A Kohlenstoff ist, dieser entweder ein Wasserstoffatom tragen oder wiederum, wie oben für X und Y definiert, substituiert sein kann. Dabei bilden diese Reste zusammen mit den Atomen, an die sie gebunden sind, einen weiteren Ring, der vorzugsweise mit den übrigen Ringen innerhalb der Struktureinheit ein konjugiertes, ungesättigtes System bildet.

Als bevorzugter, dabei entstehender Ring ist der Cyclobuten-Ring zu nennen.

Eine bevorzugte Gruppe der erfindungsgemäßen Polyselenophene stellen solche mit Struktureinheiten der allgemeinen Formeln (VII) und/oder (VIII) dar 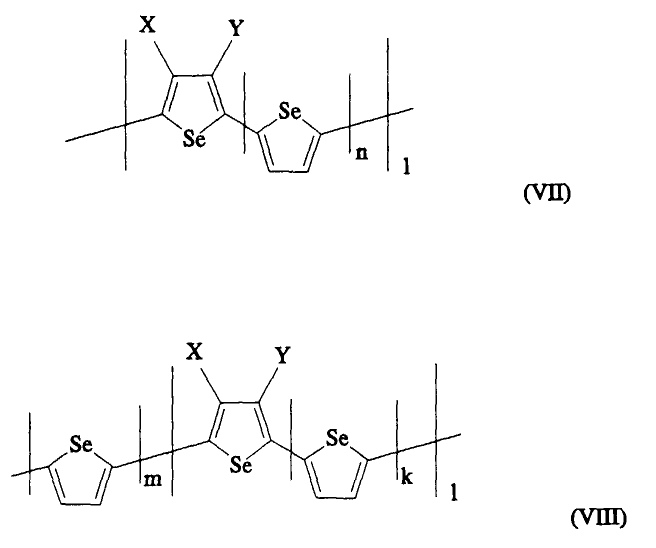 in denen X und Y wie oben definiert sind und n, m und k unabhängig voneinander eine ganze Zahl von 1 bis 10, vorzugsweise eine ganze Zahl von 1 bis 6, bedeuten, und 1 eine ganze Zahl von 1 bis 3.000, vorzugsweise 1 bis 1.000, insbesondere 1 bis 100, bedeutet. Dabei ist die alternierende Abfolge einer substituierten Selenopheneihheit und einer unsubstituierten Selenopheneinheit bevorzugt.

Besonders bevorzugte Polyselenophene stellen solche dar, in denen die Struktureinheit (II) ausgewählt wird aus der Gruppe bestehend aus den Resten der folgenden allgemeinen Formeln (IIa) bis (IIg) 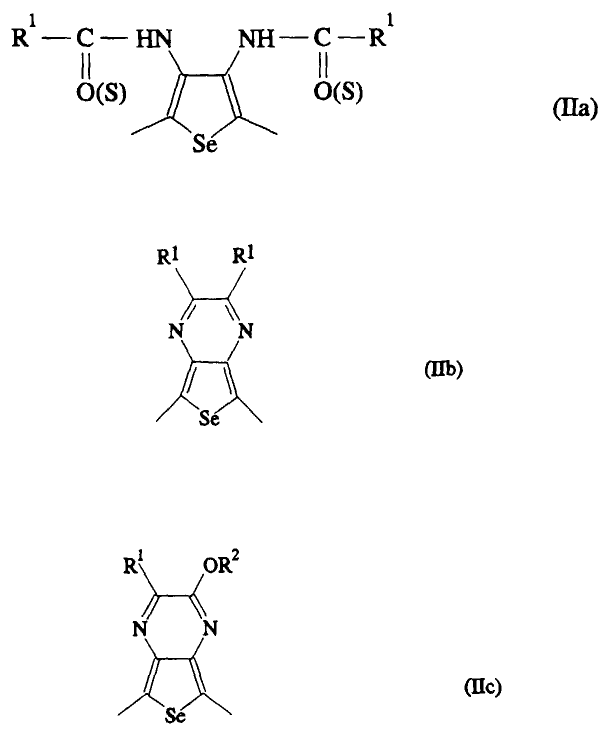 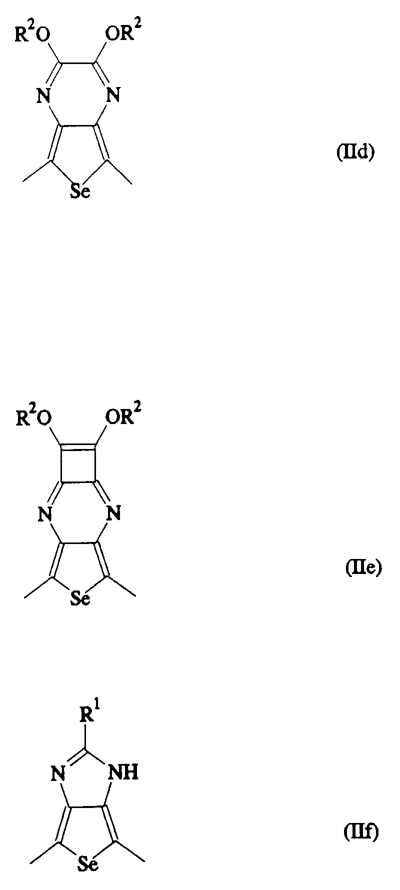 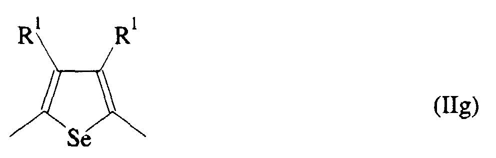
wobei R² = CH₂R¹ oder R² = CHR¹₂, R¹ = H oder wie in Anspruch 1 definiert ist.

Unter den oben genannten Struktureinheiten (II) sind die folgenden, individuell aufgelisteten Struktureinheiten besonders bevorzugt. Dabei ist es selbstverständlich, daß sich diese von den entsprechenden, in Nachbarschaft zum Selenophen-Selenatom di-substituierten Verbindungen ableiten:

3,4-Di(decyl)selenophen-, 3,4-Di(undecyl)selenophen-, 3,4-Di(dodecyl)selenophen-, 3,4-Di(tridecyl)selenophen-, 3,4-Di(tetradecyl)selenophen-, 3,4-Di(pentadecyl)selenophen-, 3,4-Di(hexadecyl)selenophen-, 3,4-Di(heptadecyl)selenophen- und 3,4-Di(octadecyl)selenophen-Struktureinheiten;
3,4-Di(decyloxy)selenophen-, 3,4-Di(undecyloxy)selenophen-, 3,4-Di(dodecyloxy)selenophen-, 3,4-Di(tridecyloxy)selenophen-, 3-4-Di(tetradecyloxy)selenophen-, 3,4-Di(pentadecyloxy)selenophen-, 3,4-Di(hexadecyloxy)selenophen-, 3,4-Di(heptadecyloxy)selenophen- und 3,4-Di(octadecyloxy)selenophen-Struktureinheiten; weiterhin bevorzugt sind Struktureinheiten, in denen die oben definierten Etherreste durch die entsprechenden Thioetherreste ersetzt sind;
3,4-Di(decyloxyethyl)selenophen-, 3,4-Di(undecyloxyethyl)selenophen-, 3,4-Di(dodecyloxyethyl)selenophen-, 3,4-Di(tridecyloxyethyl)selenophen-, 3,4-Di(tetradecyloxyethyl)selenophen-, 3,4-Di(pentadecyloxyethyl)selenophen-, 3,4-Di(hexadecyloxyethyl)selenophen-, 3,4-Di(heptadecyloxyethyl)selenophen- und 3,4-Di(octadecyloxyethyl)selenophen-Struktureinheiten;
wobei ferner Struktureinheiten bevorzugt sind, in denen sich das (die) Sauerstoffatom(e) innerhalb der oben näher definierten Alkyloxyalkyl-Gruppen in z.B. der 3-, 3,6-, 3,6,9-, 3,6,9,12-Position(en), usw., je nach Gesamtlänge des Substituenten, befindet(en), wie z.B. 3,4-Di(ethyl-2-oxydecyl)selenophen; 3,4-Di(propyl-3-oxydecyl)selenophen; 3,4-Di(butyl-4-oxydecyl)selenophen; usw.; 3,4-Di(2-(2-(decyloxy)ethoxy)ethyl)selenophen; 3,4-Di(2-(2-(undecyloxy)ethoxy)- ethyl)selenophen; 3,4-Di(2-(2-(dodecyloxy)ethoxy)ethyl)selenophen; usw., wobei die Gesamtzahl der C-Atome 22 nicht übersteigt; weiterhin bevorzugt sind Struktureinheiten, in denen das Sauerstoffatom der oben definierten Alkyloxyalkyl-Substituenten durch Schwefel ersetzt wird;
3,4-Di(cyclopentyl)selenophen-, 3,4-Di(cyclopentenyl)selenophen-, 3,4-Di(cyclohexyl)selenophen-, 3,4-Di(cyclohexenyl)selenophen-, 3,4-Di(cyclohexadienyl)selenophen-, 3,4-Di(phenyl)selenophen- und 3,4-Di(benzyl)selenophen-Struktureinheiten, wobei die oben angegebenen Substituenten wiederum durch eine oder mehrere der für R¹ definierten Gruppen substituiert sein können; 3,4-Di(decanoyl)selenophen-, 3,4-Di(undecanoyl)selenophen-, 3,4-Di(dodecanoyl)selenophen-, 3,4-Di(tridecanoyl)selenophen-, 3,4-Di(tetradecanoyl)selenophen-, 3,4-Di(pentadecanoyl)selenophen-, 3,4-Di(hexadecanoyl)selenophen-, 3,4-Di(heptadecanoyl)selenophen- und 3,4-Di(octadecanoyl)selenophen-Struktureinheiten; sowie die entsprechenden Alkanoyloxy-Struktureinheiten, wie z.B. 3,4-Di(decanoyloxy)selenophen, 3,4-Di(undecanoyloxy)selenophen, usw; weiterhin bevorzugt sind auch hier Substituenten, in denen die sich darin befindliche Carbonylgruppe durch eine Thiocarbonylgruppe ersetzt wird;
3,4-Di(decanoylamino)selenophen-, 3,4-Di(undecanoylamino)selenophen-, 3,4-Di(dodecanoylamino)selenophen-, 3,4-Di(tridecanoylamino)selenophen-, 3,4-Di(tetradecanoylamino)selenophen-, 3,4-Di(pentadecanoylamino)selenophen-, 3,4-Di(hexadecanoylamino)selenophen-, 3,4-Di(heptadecanoylamino)selenophen- und 3,4-Di(octadecanoylamino)selenophen-Struktureinheiten, wobei auch hier das Sauerstoffatom durch Schwefel ersetzt werden kann;

2,3-Dipentylseleno[3,4-b]pyrazin, 2,3-Didecylseleno[3,4-b]pyrazin-, 2,3-Diundecylseleno[3,4-b]pyrazin-, 2,3-Didodecylseleno[3,4-b]pyrazin-, 2,3-Ditridecylseleno[3,4-b]pyrazin-, 2,3-Ditetradecylseleno[3,4-b]pyrazin-, 2,3-Dipentadecylseleno[3,4-b]pyrazin-, 2,3-Dihexadecylseleno[3,4-b]pyrazin-, 2,3-Diheptadecylseleno[3,4-b]pyrazin- und 2,3-Dioctadecylseleno[3,4-b]pyrazin-Struktureinheiten;
2-Methyl-3-decyloxyseleno[3,4-b]pyrazin-, 2-Methyl-3-undecyloxyseleno[3,4-b]pyrazin-, 2-Methyl-3-dodecyloxyseleno[3,4-b]pyrazin-, 2-Methyl-3-tridecyloxyseleno[3,4-b]pyrazin-, 2-Methyl-3-tetradecyloxyseleno[3,4-b]pyrazin-, 2-Methyl-3-pentadecyloxyseleno[3,4-b]pyrazin-, 2-Methyl-3-hexadecyloxyseleno[3,4-b]pyrazin-, 2-Methyl-3-octadecyloxyseleno[3,4-b]pyrazin-, 2-Methyl-3-eicosyloxyseleno[3,4-b]pyrazin-, 2-Methyl-3-docosyloxyseleno[3,4-b]pyrazin-, 2-Ethyl-3-decyloxyseleno[3,4-b]pyrazin-, 2-Ethyl-3-undecyloxyseleno[3,4-b]pyrazin-, 2-Ethyl-3-dodecyloxyseleno[3,4-b]pyrazin-, 2-Ethyl-3-tridecyloxyseleno[3,4-b]pyrazin-, 2-Ethyl-3-tetradecyloxyseleno[3,4-b]pyrazin-, 2-Ethyl-3-pentadecyloxyseleno[3,4-b]pyrazin-, 2-Ethyl-3-hexadecyloxyseleno[3,4-b]pyrazin-, 2-Ethyl-3-octadecyloxyseleno[3,4-b]pyrazin-, 2-Ethyl-3-eicosyloxyseleno[3,4-b]-pyrazin-, 2-Ethyl-3-docosyloxyseleno[3,4-b]pyrazin-Struktureirheiten, 2-Phenyl-3-decyloxyseleno[3,4-b]pyrazin-, 2-Phenyl-3-undecyloxyseleno[3,4-b]pyrazin-, 2-Phenyl-3-dodecyloxyseleno[3,4-b]pyrazin-, 2-Phenyl-3-tridecyloxyseleno[3,4-b]pyrazin-, 2-Phenyl-3-tetradecyloxyseleno[3,4-b]pyrazin-, 2-Phenyl-3-pentadecyloxyseleno[3,4-b]pyrazin-, 2-Phenyl-3-hexadecyloxyseleno[3,4-b]pyrazin-, 2-Phenyl-3-heptadecyloxyseleno[3,4-b]pyrazin-, 2-Phenyl-3-octadecyloxyseleno[3,4-b]pyrazin-, 2-Phenyl-3-eicosyloxyseleno[3,4-b]pyrazin- und 2-Phenyl-3-docosyloxyseleno[3,4-b]pyrazin-Struktureinheiten, wobei auch hier das Sauerstoffatom im Substituenten durch Schwefel ersetzt werden kann;
2,3-Di(decyloxy)seleno[3,4-b]pyrazin-, 2,3-Di(undecyloxy)seleno[3,4-b]pyrazin-, 2,3-Di(dodecyloxy)seleno[3,4-b]pyrazin-, 2,3-Di(tridecyloxy)seleno[3,4-b]pyrazin-, 2,3-Di(tetradecyloxy)seleno[3,4-b]pyrazin-, 2,3-Di(pentadecyloxy)seleno[3,4-b]pyrazin-, 2,3-Di(hexadecyloxy)seleno[3,4-b]pyrazin-, 2,3-Di(heptadecyloxy)seleno[3,4-b]pyrazin-, 2,3-Di(octadecyloxy)seleno[3,4-b]pyrazin-, 2,3-Di(eicosyloxy)seleno[3,4-b]pyrazin- und 2,3-Di(docosyloxy)seleno[3,4-b]pyrazin-Struktureinheiten; weiterhin bevorzugt sind Struktureinheiten, in denen die oben definierten Etherreste durch die entsprechenden Thioetherreste ersetzt sind;
2,3-Di(decyloxyethyl)seleno[3,4-b]pyrazin-, 2,3-Di(undecyloxyethyl)seleno[3,4-b]pyrazin-, 2,3-Di(dodecyloxyethyl)seleno[3,4-b]pyrazin-, 2,3-Di(tridecyloxyethyl)seleno[3,4-b]pyrazin-, 2,3-Di(tetradecyloxyethyl)seleno[3,4-b]pyrazin-, 2,3-Di(pentadecyloxyethyl)seleno[3,4-b]pyrazin-, 2,3-Di(hexadecyloxyethyl)seleno[3,4-b]pyrazin-, 2,3-Di(heptadecyloxyethyl)seleno[3,4-b]pyrazin- und 2,3-Di(octadecyloxyethyl)seleno[3,4-b]pyrazin-Struktureinheiten; wobei ferner Struktureinheiten bevorzugt sind, in denen sich das (die) Sauerstoffatom(e) innerhalb der oben näher definierten Alkyloxyalkyl-Gruppen z.B. in der 3-, 3,6-, 3,6,9-, 3,6,9,12-Position, usw., je nach Gesamtlänge des Substituenten, befindet; wie z.B. 2,3-Di(ethyl-2-oxydecyl)seleno[3,4-b]pyrazin; 2,3-Di(propyl-3-oxydecyl)seleno[3,4-b]pyrazin; 2,3-Di(butyl-4-oxydecyl)seleno[3,4-b]pyrazin; usw.; 2,3-Di(2-(2-(decyloxy)ethoxy)ethyl)seleno[3,4-b]pyrazin; 2,3-Di(2-(2-(undecyloxy)ethoxy)ethyl)seleno[3,4-b]pyrazin; 2,3-Di(2-(2-(dodecyloxy)ethoxy)ethyl)seleno[3,4-b]pyrazin; usw., wobei jeweils die Gesamtzahl der C-Atome 22 nicht übersteigt; weiterhin bevorzugt sind Struktureinheiten, in denen das Sauerstoffatom der oben definierten Alkyloxyalkyl-Substituenten durch Schwefel ersetzt wird;
2,3-Di(cyclopentyl)seleno[3,4-b]pyrazin-, 2,3-Di(cyclopentenyl)seleno[3,4-b]pyrazin-, 2,3-Di(cyclohexyl)seleno[3,4-b]pyrazin-, 2,3-Di(cyclohexenyl)seleno[3,4-b]pyrazin-, 2,3-Di(cyclohexadienyl)seleno[3,4-b]pyrazin-, 2,3-Di(phenyl)seleno[3,4-b]pyrazin- und 2,3-Di(benzyl)seleno[3,4-b]pyrazin-Struktureinheiten, wobei die oben angegebenen Substituenten wiederum durch eine oder mehrere der für R¹ definierten Gruppen substituiert sein können;
5,6-Di(decyloxy)cyclobuta[b]seleno[3,4-e]pyrazin-, 5,6-Di(undecyloxy)cyclobuta[b]seleno[3,4-e]pyrazin-, 5,6-Di(dodecyloxy)cyclobuta[b]seleno[3,4-e]pyrazin-, 5,6-Di(tridecyloxy)cyclobuta[b]seleno[3,4-e]pyrazin-, 5,6-Di(tetradecyloxy)cyclobuta[b]seleno[3,4-e]pyrazin-, 5,6-Di(pentadecyloxy)cyclobuta[b]seleno[3,4-e]pyrazin-, 5,6-Di(hexadecyloxy)cyclobuta[b]seleno[3,4-e]pyrazin-, 5,6-Di(heptadecyloxy)cyclobuta[b]seleno[3,4-e]pyrazin- und 5,6-Di(octadecyloxy)cyclobuta[b]seleno[3,4-e]pyrazin-Struktureinheiten; weiterhin bevorzugt sind Struktureinheiten, in denen die oben definierten Etherreste durch die entsprechenden Thioetherreste ersetzt sind;
5,6-Di(cyclopentyloxy)cyclobuta[b]seleno[3,4-e]pyrazin-, 5,6-Di(cyclopentenyloxy)cyclobuta[b]seleno[3,4-e]pyrazin-, 5,6-Di(cyclohexyloxy)cyclobuta[b]seleno[3,4-e]pyrazin-, 5,6-Di(cyclohexenyloxy)cyclobuta[b]seleno[3,4-e]pyrazin-, 5,6-Di(cyclohexadienyloxy)cyclobuta[b]seleno[3,4-e]pyrazin-, 5,6-Di(phenyl)cyclobuta[b]seleno[3,4-e]pyrazin- und 5,6-Di(benzyl)cyclobuta[b]seleno[3,4-e]pyrazin-Struktureinheiten, wobei die oben angegebenen Substituenten wiederum durch eine oder mehrere der für R¹ definierten Gruppen substituiert sein können;
2-Decyl-1H-seleno[3,4-d]imidazol-, 2-Undecyl-1H-seleno[3,4-d]imidazol-, 2-Dodecyl-1H-seleno[3,4-d]imidazol-, 2-Tridecyl-1H-seleno[3,4-d]imidazol-, 2-Tetradecyl-1H-seleno[3,4-d]imidazol-, 2-Pentadecyl-1H-seleno[3,4-d]imidazol-, 2-Hexadecyl-1H-seleno[3,4-d]imidazol-, 2-Heptadecyl-1H-seleno[3,4-d]imidazol- und 2-Octadecyl-1H-seleno[3,4-d]imidazol-Struktureinheiten;
2-Cyclopentyl-1H-seleno[3,4-d]imidazol-, 2-Cyclopentenyl-1H-seleno[3,4-d]imidazol-, 2-Cyclohexyl-1H-seleno[3,4-d]imidazol-, 2-Cyclohexenyl-1H-seleno[3,4-d)imidazol-, 2-Cyclohexadienyl-1H-seleno[3,4-d]imidazol-, 2-Phenyl-1H-seleno[3,4-d]imidazol- und 2-Benzyl-1H-seleno[3,4-d]imidazol-Struktureinheiten; 2-Burylthio-1H-seleno[3,4-d]imidazol-, 2-Pentylthio-1H-seleno[3,4-d]imidazol-, 2-Hexylthio-1H-seleno[3,4-d]imidazol-, 2-Heptylthio-1H-seleno[3,4-d]imidazol-, 2-Octylthio-1H-seleno[3,4-d]imidazol-, 2-Nonylthio-1H-seleno[3,4-d]-imidazol-, 2-Decylthio-1H-seleno[3,4-d]imidazol-, 2-Undecylthio-1H-seleno[3,4-d]imidazol-, 2-Dodecylthio-1H-seleno[3,4-d]imidazol-, 2-Tridecylthio-1H-seleno[3,4-d]imidazol-, 2-Tetradecylthio-1H-seleno[3,4-d]imidazol-, 2-Pentadecylthio-1H-seleno[3,4-d]imidazol-, 2-Hexadecylthio-1H-seleno[3,4-d]- imidazol-, 2-Heptadecylthio-1H-seleno[3,4-d]imidazol-, 2-Octadecylthio-1H-seleno[3,4-d]imidazol-Struktureinheiten, wobei die oben angegebenen Substituenten wiederum durch eine oder mehrere der für R¹ definierten Gruppen substituiert sein können.

Die Anteile der oben definierten Struktureinheiten (I) und (II) in den erfindungsgemäßen Polyselenophenen betragen ungefähr 1 bis ungefähr 99 Mol-% (I) und ungefähr 99 bis ungefähr 1 Mol-% (II), vorzugsweise ungefähr 30 bis ungefähr 70 Mol-% (I) und ungefähr 70 bis ungefähr 30 Mol-% (II), und insbesondere ungefähr 50 Mol-% (I) und ungefähr 50 Mol-% (II), wobei sich die Anteile dieser beiden Struktureirheiten jeweils zu 100 Mol-% addieren. Vorzugsweise ist die Abfolge der beiden Struktureinheiten (I) und (II) alternierend.

Das Gewichtsmittel des Molekulargewichts (M_{w}) der erfindungsgemäß hergestellten Polyselenophene, gemessen durch Gelpermeationschromatographie unter Verwendung von Polystyrol als Standard beträgt im allgemeinen ungefähr 1.000 bis ungefähr 500.000, bevorzugt ungefähr 10.000 bis ungefähr 250.000, und insbesondere ungefähr 30.000 bis ungefähr 80.000. Die Uneinheitlichkeit der Molekulargewichtsverteilung, d.h. der Quotient aus dem Gewichtsmittel des Molekulargewichts und dem Zahlenmittel des Molekulargewichts (M_{w}/Mₙ), beträgt ungefähr 2 bis ungefähr 4, bevorzugt ungefähr 2 bis ungefähr 3, und insbesondere ungefähr 2 bis ungefähr 2,5.

Die erfindungsgemäßen Polyselenophene können mittels aller für die Polymerisation von Pyrrolen oder Thiophenen geeigneten Umsetzungen polymerisiert werden, wie z.B. mittels chemischer oder elektrochemischer Oxidation; einige der Polyselenophene, die beispielsweise Alkyl-, Alkoxy-, Acylamino- oder Alkyloxyalkyl-Substituenten tragen, können auch durch katalytische Kupplung von metallorganischen Reagenzien (meistens Grignard-Reagenzien) umgesetzt werden. Bezüglich der Details dieser Umsetzung wird auf die in der Einleitung erwähnte PCT/EP97/01140 und die darin referierte Literatur verwiesen.

Die bevorzugten Verfahren zur Herstellung der erfindungsgemäßen Polyselenophene stellen jedoch die Umsetzung nach Stille sowie die Umsetzung nach Suzuki (s. A. Suzuki et al. "Stereoselective synthesis of arylated (E)-alkanes by the reaction of alk-1-enylboranes with aryl halides in the presence of palladium catalyst", J. C. S. Chem. Comm., 1979, S. 866-867) dar, die im folgenden näher erläutert werden sollen.

Bei der Suzuki-Umsetzung werden 2,5-Dihalogen- oder 2,5-Triflat-substituierte Selenophene, die der erfindungsgemäßen Struktureinheit (II) entsprechen, mit Selenophendiborsäure oder Selenophendiborsäureestern in Anwesenheit einer Base, vorzugsweise Natriumethoxid, und eines Palladium-Komplexes der Struktur PdL₄ (L = Ligand), vorzugsweise Pd(PPh₃)₄, umgesetzt. 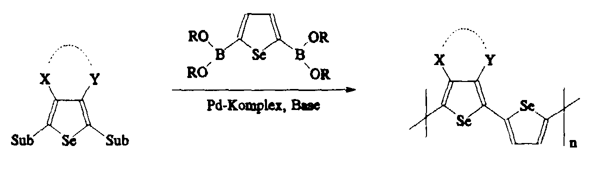
- Sub: = Halogen oder Triflat
- X,Y: = wie oben definiert
- R: = H oder Alkyl

Als bevorzugte Borane werden bei dieser Umsetzung Selenophen-2,5-diborsäure und "ihre" Ester eingesetzt. Bezüglich weiterer Details dieser Umsetzung wird auf die oben zitierte Literaturstelle verwiesen.

Selbstverständlich können im Rahmen der Suzuki-Umsetzung die Diborsäure(ester)derivate der den obigen Struktureinheiten (II) entsprechenden Selenophenderivate mit 2,5-Dihalogen- oder 2,5-Triflat-substituiertem Selenophen, das der Struktureinheit (I) entspricht, umgesetzt werden.

Das insbesondere bevorzugte Verfahren zur Herstellung der erfindungsgemäßen Polyselenophene stellt jedoch die bereits eingangs erwähnte Stille-Umsetzung dar. In dieser Umsetzung werden 2,5-Dihalogenselenophen bzw. 2,5-Ditriflatselenophen und an den dem Selen benachbarten Kohlenstoffatomen Bis(trialkylzinn)-substituierte Selenophenderivate, die der oben definierten Struktureinheit (II) entsprechen, oder 2,5-Bis(trialkylzinn)selenophen und an den dem Selen benachbarten Kohlenstoffatomen Bis(halogen)- oder Bis(triflat)-substituierte Selenophenderivate, die der oben definierten Struktureinheit (II) entsprechen, nach dem folgenden Schema miteinander in geeigneten Lösungsmitteln in Anwesenheit von geeigneten Pd(0)- oder Pd(II)-Komplexen oder Salzen als Katalysator umgesetzt. 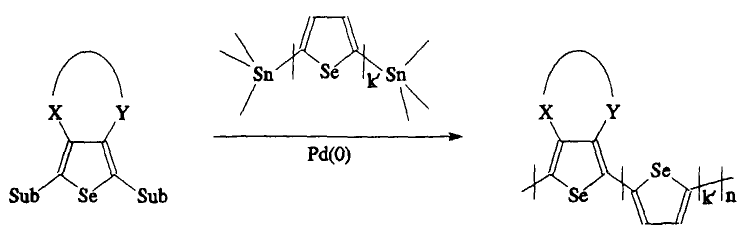
- Sub: = Halogen oder Triflat
- k': = 1,2,3,...10

Unter den in obigem Schema erwähnten Substituenten "Sub" werden Halogen-substituierte Derivate bevorzugt eingesetzt. Sofern Bis(triflat)-, d.h. (CF₃SO₃)-substituierte Edukte eingesetzt werden, kann z.B. LiCl zur Verbesserung der Reaktivität zugesetzt werden.

Aufgrund der milden Bedingungen ihrer Durchführung toleriert diese Reaktion alle Arten von Substituenten, wie z.B. Amin-, Acyl-, Ester-, Ether- und Nitro-Gruppen.

Als Katalysatoren werden Pd(II)- oder Pd(0)-Komplexe eingesetzt. Als bevorzugte Katalysatoren sind insbesondere die folgenden zu nennen: Tris(dibenzylidenaceton)dipalladium (Pd₂dba₃), Pd(Ph₃P)₂Cl₂, wobei "Ph" für C₆H₅ steht, und Pd(Ph₃P)₄. Bei der Verwendung von Tris(dibenzylidenaceton)dipalladium können verschiedene Liganden (L) zugesetzt werden, wobei der katalytisch wirksame Katalysator PdL₄ in situ durch Ligandenaustausch zwischen dem schwach koordinierten Pd₂dba₃ und dem oder den Liganden gebildet wird. Als Liganden sind dabei PPh₃, AsPh₃, [2-(CH₃)C₆H₄]₃P, P(OPh)₃ und (2-Furyl)₃P verwendet werden. Die Menge des eingesetzten Katalysators beträgt ungefähr 2 bis ungefähr 10 Mol-%, vorzugsweise ungefähr 2 bis ungefähr 5 Mol-%, bezogen auf die eingesetzte Menge des Bis(trialkylzinn)-substituierten Edukts.

Obwohl im allgemeinen alle Lösungsmittel, die in der Lage sind, die eingesetzten Edukte und Katalysatoren in Lösung zu halten, eingesetzt werden können, stellen DMF, NMP und cyclische Ether, wie z.B. THF und Dioxan, die am meisten geeigneten Lösungsmittel dar. Unter diesen werden DMF und THF bevorzugt eingesetzt.

Die Umsetzung wird im allgemeinen bei Temperaturen zwischen Raumtemperatur und dem Siedepunkt des Lösungsmittels durchgeführt, wobei Temperaturen von ungefähr 50 °C bis ungefähr 100 °C bevorzugt sind. Die Reaktionsdauer variiert je nach verwendeten Edukten und/oder Katalysatoren zwischen 1 Tag und 1 Monat, vorzugsweise 1 Tag bis 1 Woche.

Sofern Copoly- oder Cooligomere mit Thiophen- und oder anderen konjugierten organischen Einheiten hergestellt werden sollen, wird analog vorgegangen.

Durch diese relativ milde Umsetzung ist es möglich, Polyselenophene mit folgenden vorteilhaften Eigenschaften herzustellen:
- Die erhaltenen Selenophene weisen lediglich geringe oder keine strukturellen Defekte, Dotierung oder Überoxidation auf;
- es ist möglich, eine große Vielzahl funktioneller Gruppen einschließlich Akzeptoren, Donatoren und π-Systemen mit mehreren Bindungen zwischen Kohlenstoff und einem Heteroatom einzusetzen; und
- es ist möglich, streng alternierende Copolyselenophene mit verschieden substituierten Ringen, beispielsweise die Umsetzung von in 3- und 4-Stellung substituierten Selenophenen mit unsubstituierten Selenophenen durchzuführen, was mit den bislang allgemein verwendeten Verfahren zur Polyselenophenherstellung nur äußerst schwierig möglich war.

Obwohl auch die Suzuki-Umsetzung die oben aufgeführten Vorteile gegenüber den herkömmlichen Methoden aufweist, wird im Rahmen der vorliegenden Erfindung die Stille-Umsetzung insbesondere bevorzugt, da sie - im Unterschied zu Suzuki-Umsetzung - keine zusätzliche Base benötigt.

Ferner stellt die vorliegende Erfindung Verbindungen der allgemeinen Formeln (IXa) bis (IXc) 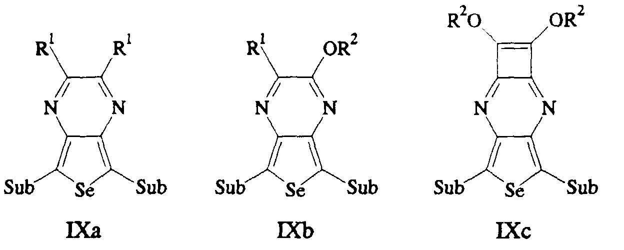 in denen "Sub" Wasserstoff (H), Brom (Br), Chlor (Cl), Iod (I), Triflat (CF₃SO₃) oder Trialkylzinn ist, und R¹ wie oben definiert ist, und R² CH₂R¹ oder CHR¹₂ ist, bereit.

Außerdem werden N,N-substituierte 3,4-Diamino-2,5-disubstituierte (vorzugsweise -dihalogen- oder ditriflat)selenophene der allgemeinen Formel (X) 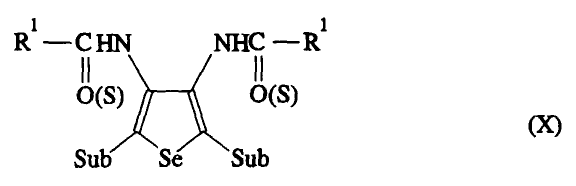 in der "Sub" und R¹ wie oben definiert sind, bereitgestellt.

Als Substituenten "Sub" sind die oben erwähnten Halogenreste bevorzugt, weiter bevorzugt sind Iod oder Brom. Bezüglich bevorzugter Reste R¹ wird auf die weiter oben gegebene Auflistung verwiesen.

Obwohl diese Monomerklasse im allgemeinen nach herkömmlichen Verfahren zur Herstellung solcher Verbindungen dargestellt werden können, wird das bei den Beispielen erläuterte allgemeine Verfahren zur Herstellung der erfindungsgemäßen Monomere bevorzugt angewandt.

Ein weiterer Aspekt der vorliegenden Erfindung stellt die Bereitstellung von 2,5-Bis(trialkylzinn)selenophen (Formel XIa), 5,5'-Bis(trialkylzinn)-2,2'-diselenophens (Formel XIb) sowie des 5,5''-Bis(trialkylzinn)-2,2':5',2''-Terselenophenen der Formel (XIc), sowie des 2-(Trialkylzinn)selenophens (Formel XId), die ebenfalls zur Synthese von Oligo- und Polyselenophenen verwendet werden können, dar, 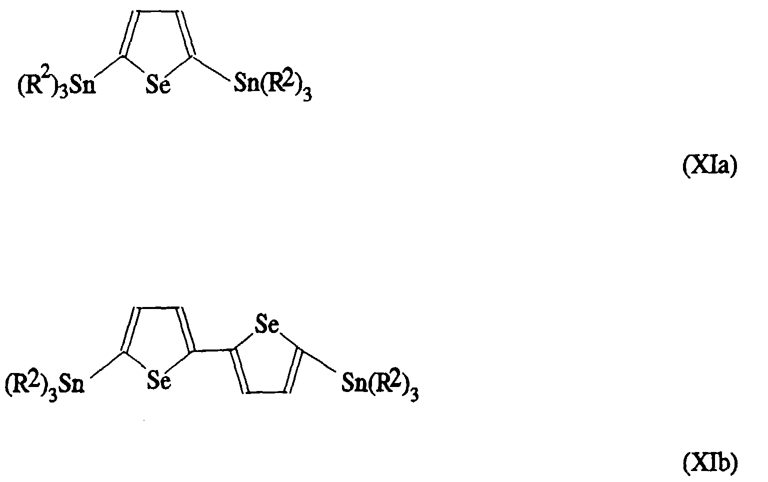 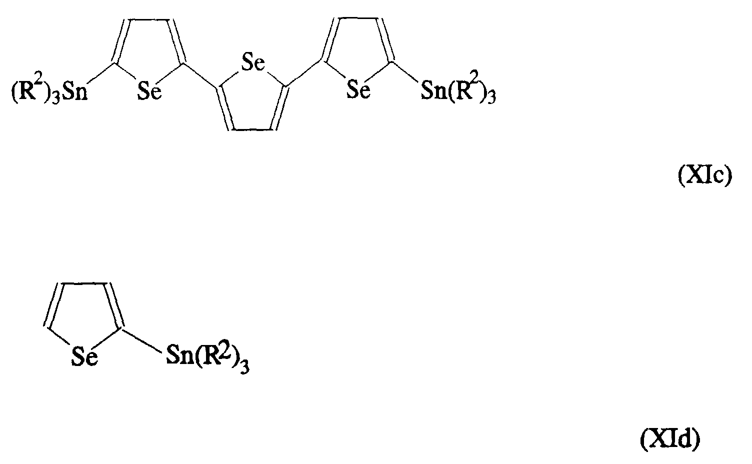 wobei R² eine Alkylgruppe, vorzugsweise eine Methyl- oder Butylgruppe, darstellt. Die Herstellung dieser Selenophene ist in den Beispielen beschrieben.

Ferner werden erfindungsgemäß das Di- und Trimere des Selenophens, d. h. 2,2'-Biselenophen und 2,2':5',2''-Terselenophens bereitgestellt.

Dünne Filme der erfindungsgemäßen Polyselenophene können durch Vergießen von sorgfältig filtrierten oder zentrifugierten Lösungen in Chloroform oder Trichlorethylen auf ein Glassubstrat hergestellt werden. Durch dieses Verfahren können homogene Filme mit einer Dicke von bis zu 300 nm erhalten werden. Die optische Qualität der Filme ist hoch und weist keine nennenswerte Streuung des Lichts auf.

Die hierin beschriebenen Polyselenophene können entweder undotiert oder dotiert zur antistatischen Ausrüstung von den elektrischen Strom nicht oder nur schlecht leitenden Substanzen, als elektrisch leitfähige Folien, als Halbleiter-Folien, als Zusatzstoff für aktive Elektroden, für LED's, als organische Transistoren und Kondensatoren verwendet werden. Dabei werden diese Copolymere in üblicher Weise verarbeitet und ggf. mit bekannten, für die jeweilige Anwendung erforderlichen Zusatz- und Konfektionierungssubstanzen vermischt bzw. nach bekannten Verfahren verarbeitet.

Darüber hinaus stellt die vorliegende Erfindung noch ein Verfahren zur antistatischen Ausrüstung eines den elektrischen Strom nicht oder nur schlecht leitenden Substrats durch Aufbringen einer Schicht enthaltend ein elektrisch leitfähiges organisches Polymer auf die Oberfläche der Substrate bereit, das dadurch gekennzeichnet ist, daß man auf die Oberfläche des Substrats durch Polymerisation eine Schicht mindestens eines Polyselenophens erzeugt, das die oben definierten Struktureinheiten der allgemeinen Formeln (I) und/oder (II) enthält. Diese oben genannte Schicht kann dabei auch ausschließlich aus dem oben definierten leitfähigen organischen Polymer bestehen.

Ferner stellt die vorliegende Erfindung ein elektrisch leitfähiges Material bereit, das mindestens ein Polyselenophen gemäß der vorliegenden Erfindung enthält.
Die vorliegende Erfindung soll nunmehr an einigen ausgewählten Beispielen weiter erläutert werden.

### BEISPIELE

### Beispiel 1

2,5-Bis(trimethylzinn)selenophen (A) wurde direkt ausgehend vom Selenophen 1 über das 2,5-Dilithiumderivat 2 erhalten. Verbindung 2 wurde gemäß B.J. Wakefield, "Organolithium methods", Academic Press, 1988, S. 47 - 48 (der russischen Übersetzung) und der darin zitierten Literatur, in der die Umsetzung für das entsprechende Thiophen beschrieben wird, durch Umsetzung mit BuLi/Hexan mit TMEDA unter Rückfluß hergestellt. Die Umsetzung wird durch das untenstehende Schema 1 veranschaulicht: 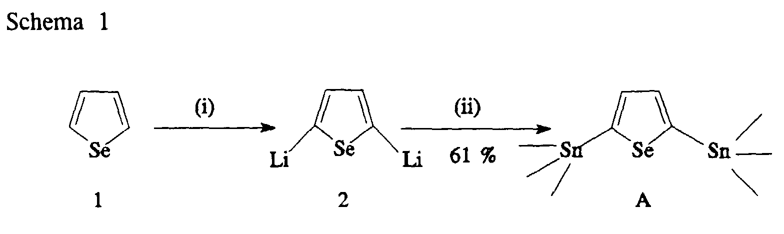
i: 2,5 Equivalente von BuLi/TMEDA; Hexan, Rückfluß
ii: (CH₃)₃SnCl, Hexan, 0°C, Raumtemperatur

Eine analytisch saubere Probe der Verbindung A wurde nach zweimaligen Urnkristallisieren in Hexan erhalten. Die Struktur (und Reinheit) der Verbindung A wurde durch ¹H-NMR-Spektroskopie bestätigt.

Die Verbindung A besaß einen Schmelzpunkt von 123 bis 124°C (Hexan).
¹H-NMR(200MHz, CDCl₃, δ, ppm):
   0,38 s (CH₃), 0,38 d (CH₃, J(¹¹⁷Sn-¹H) = J(¹¹⁹Sn-¹H) = 56,7 Hz), Gesamtintensität beider Signale: 9H), 7,70 s (H^{ar}); bedingt durch eine Spin-Spin-Kopplung (³J) mit ¹¹⁷Sn/¹¹⁹Sn und ⁷⁷Se wurde ein symmetrisches Multiplet um 7,70 ppm mit einer Gesamtintensität, die 1H entsprach, beobachtet.

### Beispiel 2

Das 2,2'-Biselenophen 6 wurde, ausgehend von 2-Iodselenophen 3 und 2-(Trimethylzinn)selenophen 5 erhalten. 2-Iodselenophen wurde wie in Yurjev et al, Russ. J. of General Chem., 26 (1956), S. 3154 beschrieben, erhalten.

2-(Trimethylzinn)selenophen 5 wurde durch Quenchen von 2-Lithiumselenophen mit Trimethylzinnchlorid erhalten. Das Monolithiumderivat wurde selektiv ausgehend von Selenophen durch Umsetzung von einem Equivalent Butyllitium in Hexan in Gegenwart von TMEDA (N,N'N',N'-Tetramethylethylendiamin) erhalten.

Die Stille-Reaktion zwischen 2-Iod- und 2-(Trimethylzinn)selenophen lieferte das dimere 2,2'-Biselenophen 6 als Hauptprodukt. Die Ausbeute an Verbindung 6 nach Reinigung derselben über Kieselgel und Abtrennen des Trimers 9 betrug 69% bezogen auf die eingesetzte Menge der Verbindung 3.

Im untenstehenden Schema 2 wird die oben beschriebene Umsetzung nochmals erläutert. Die Verbindung 6 besitzt einen Schmelzpunkt von 36 bis 38°C (Hexan).
¹H-NMR(200MHz, CDCl₃, δ, ppm):
   7,3 m (2 H, H^{b} + H^{c}), 7.94 d (1 H, H³, J_{ab} 4,5 Hz); ferner wurde ein Dublett von Dubletts (dd) mit niedriger Intensität zentriert um 7,94 ppm beobachtet, ²J(⁷⁷Se-H) = 47 Hz, J_{ab} = 5,4 Hz, Integralintensität dieses dd: 6% der Intensität des Hauptsignals, das dem natürlichen Gehalt an ⁷⁷Se (7,6) entspricht.

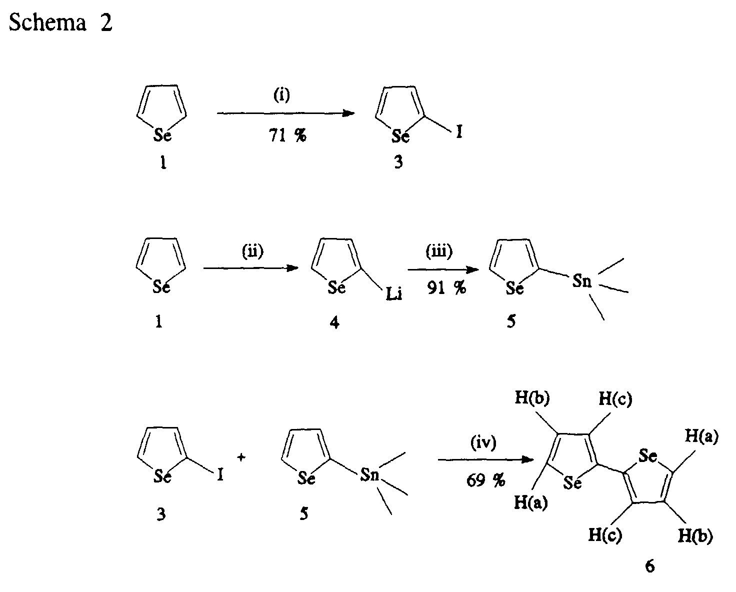

i: I₂/HgO, Benzol
ii: BuLi/TMEDA, Hexan, 0°C - Raumtemperatur (RT)
iii: (CH₃)₃SnCl, Hexan, 0°C - RT
iv: 5 mol-% Pd(Ph₃P)₂Cl₂, THF, Rückfluß

Wie in Schema 3 unten dargestellt, wurde Verbindung 6 nochmals ausgehend von 2-Bromselenophen 7, das gemäß H. Suginome, S. Umezawa, Bull. Chem. Soc., 11 (Nr. 3), S. 157 - 167 (1936) hergestellt wurde, in einer Ausbeute von ungefähr 20% bezogen auf die eingesetze Menge der Verbindung 7 erhalten. 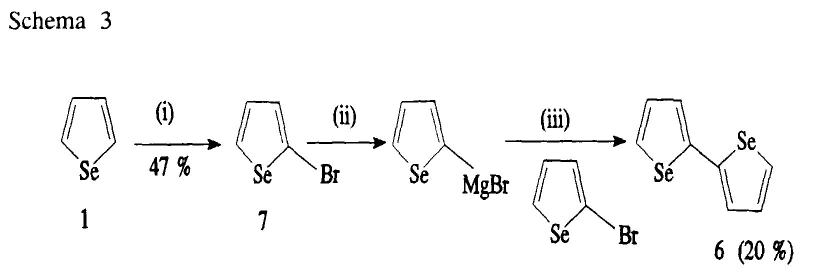
i: Br₂/CS₂, 0°C - RT
ii: Mg, THF, Rückfluß
iii: Ni(dppp)Cl₂, THF, Rückfluß

### Beispiel 3

Ausgehend von 2-Iodselenophen 3 und 2,5-Bis(trimethylzinn)selenophen A wurde das 2,2':5',2''-Terselenophen 9 gemäß untenstehendem Schema 4 erhalten

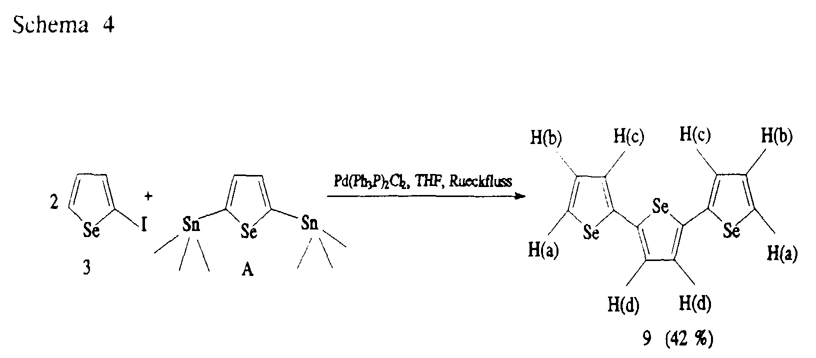

Verbindung 9 wurde in 42%-iger Ausbeute erhalten und über Kieselgel gereinigt. Verbindung 9 besaß einen Schmelzpunkt von 169 bis 171°C.
¹H-NMR(200MHz, CDCl₃, δ, ppm):
   7,14 s (2 H, H^{d}), 7,24 m (4 H, H^{b} + H^{c}), 7,88 d (2 H, H^{a}, J_{ab} 5 Hz); ferner wurde ein Dublett von Dubletts (dd) mit niedriger Intensität zentriert um 7,88 ppm beobachtet, ²J(⁷⁷Se-H) = 47 Hz, J_{ab} = 6,3 Hz.

Die anschließenden Umsetzung der Verbindung 9 zur Verbindung C wurde gemäß dem untenstehenden Schema 5 durchgeführt, wobei Verbindung C in 24%-iger Ausbeute, bezogen auf die Ausgangsverbindung 9 erhalten wurde. Zur Reinigung wurde das erhaltenen Reaktionsprodukt in kaltem Pentan gewaschen. Verbindung C zersetzt sich unter Erwärmen bei einer Temperatur von ungefähr 70°C.
¹H-NMR (200 MHz, CDCl₃, δ, ppm): 0,38 s (CH³), 0,38 d (CH₃, J(¹¹⁷Sn-¹H) = J¹¹⁹Sn-¹H) = 56,3 Hz), Gesamtinensität der beiden Signale: 18H ), 7,13 s (2H, H^{c}), 7,32 d (2 H, H^{a}/H^{b}, J 3,6 Hz), 7,37 d (2H, H^{b}/H^{a}, J 3,6 Hz).

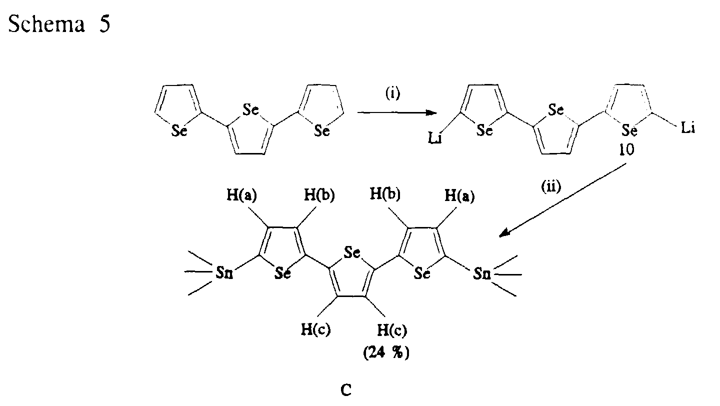

i: BuLi, TMEDA, THF, -10°C - RT
ii: (CH₃)₃SnCl, THF

### Beispiel 4

2,5'-Bis(trialkylzinn)-5,2'-diselenophen (B) 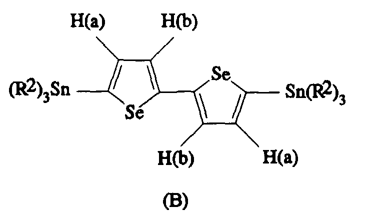 wurde analog Beispiel 3 ausgehend vom 2,2'-Biselenophen 6 in der gleichen Weise wie für das Trimere C in Schema 5 angegeben hergestellt.

Verbindung B besaß einen Schmelzpunkt von 113 bis 115°C (Hexan).
¹H-NMR (200 MHz, CDCl₃, δ, ppm): 0,38 s (CH₃), 0,38 d (CH₃, J(¹¹⁷Sn-¹H) = J(¹¹⁹Sn-¹H) = 56,2 Hz), Gesamtintensität der beiden Signale: 9H), 7,36 m (2H, H^{ar)}

## Patentansprüche

1. Polyselenophen, enthaltend eine oder mehrere Struktureinheiten der allgemeinen Formeln (I) oder (II) oder (I) und (II) 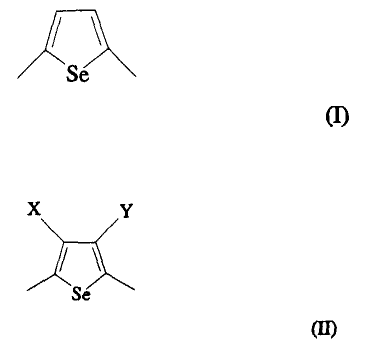 wobei
X und Y unabhängig voneinander gleich oder verschieden sein können und Wasserstoff, mit der Maßgabe, daß nicht sowohl X als auch Y Wasserstoff ist; eine lineare oder verzweigtkettige C₁- bis C₂₂-Alkylgruppe; eine lineare oder verzweigtkettige C₁- bis C₂₂-Alkoxygruppe; eine lineare oder verzweigtkettige C₁- bis C₂₂-Alkyloxyalkylgruppe; eine lineare oder verzweigtkettige C₁- bis C₂₂-Acylgruppe; eine lineare oder verzweigtkettige C₁- bis C₂₂-Thioacylgruppe; eine lineare oder verzweigtkettige C₁- bis C₂₂-Thioacyloxygruppe; eine lineare oder verzweigtkettige C₁- bis C₂₂-Acyloxygruppe; eine C₅- bis C₈-Cycloalkylgruppe, eine C₆- bis C₁₈-Arylgruppe oder eine C₅- bis C₈-heterocyclische Gruppe, die jeweils wiederum mit einer oder mehreren linearen oder verzweigtkettigen C₁- bis C₂₂-Alkylgruppe(n), einer oder mehreren linearen oder verzweigtkettigen C₁- bis C₂₂-Alkoxygruppe(n), einer oder mehreren linearen oder verzweigtkettigen C₁- bis C₂₂-Alkyloxyalkylgruppe(n), einer oder mehreren linearen oder verzweigtkettigen C₁- bis C₂₂-Acylgruppe(n) oder einer oder mehreren linearen oder verzweigtkettigen C₁- bis C₂₂-Thioacylgruppe(n) substituiert sein können; NO₂; oder NHR¹, wobei R¹ gleich oder verschieden sein kann und jeweils Wasserstoff, eine lineare oder verzweigtkettige C₁- bis C₂₂-Alkylgruppe, eine lineare oder verzweigtkettige C₁- bis C₂₂-Alkoxygruppe, eine lineare oder verzweigtkettige C₁- bis C₂₂-Alkyloxyalkylgruppe, eine lineare oder verzweigtkettige C₁- bis C₂₂-Acylgruppe oder eine lineare oder verzweigtkettige C₁- bis C₂₂-Thioacylgruppe ist, bedeuten, oder X und Y zusammen mit den Atomen, an die sie gebunden sind, ein Kohlenstoff enthaltendes Ringsystem bilden, das neben Kohlenstoff Stickstoff (N)-, Sauerstoff (O)-, Schwefel (S)- oder Phosphor (P)-Heteroatome oder Mischungen zweier oder mehrerer dieser Heteroatome aufweist, wobei
dieses Ringsystem wiederum an dem (den) Kohlenstoffatom(en), dem (den) Stickstoffatom(en) oder dem (den) Phosphoratom(en) jeweils mit einer Gruppe Z substituiert sein kann, in der jedes Z unabhängig voneinander eine Gruppe wie oben für X und Y definiert bedeutet, oder zwei benachbarte Gruppen Z zusammen einen durch eine der folgenden allgemeinen Formeln (III) bis (VI) dargestellten Rest bilden 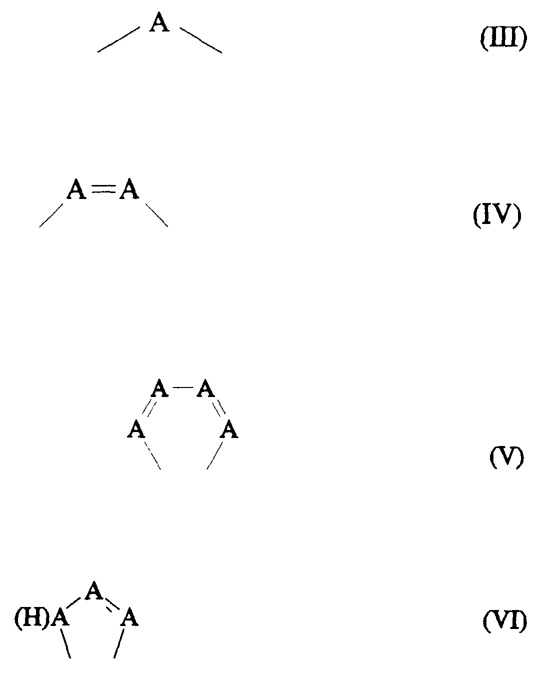 in denen A Kohlenstoff (C), Stickstoff (N), Phosphor (P) oder Mischungen zweier oder mehrerer dieser Atome bedeutet,
wobei, sofern A Kohlenstoff ist, jedes dieser A entweder ein Wasserstoffatom trägt oder wiederum wie oben für X und Y definiert, substituiert sein kann.

2. Polyselenophen nach Anspruch 1, enthaltend Struktureinheiten der allgemeinen Formeln (VII) oder (VIII) 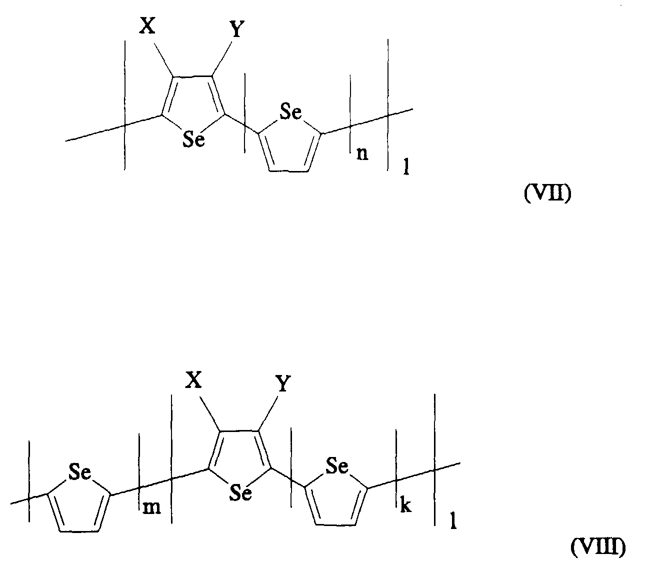
in denen X und Y wie in Anspruch 1 definiert sind,
n, m und k jeweils unabhängig voneinander eine ganze Zahl von 1 bis 10 bedeuten, und
1 eine ganze Zahl von 1 bis 3000 bedeutet.

3. Polyselenophen nach Anspruch 1 oder 2, in dem die Struktureinheit (II) ausgewählt wird aus der Gruppe bestehend aus den Resten der folgenden allgemeinen Formeln (IIa) bis (IIg) 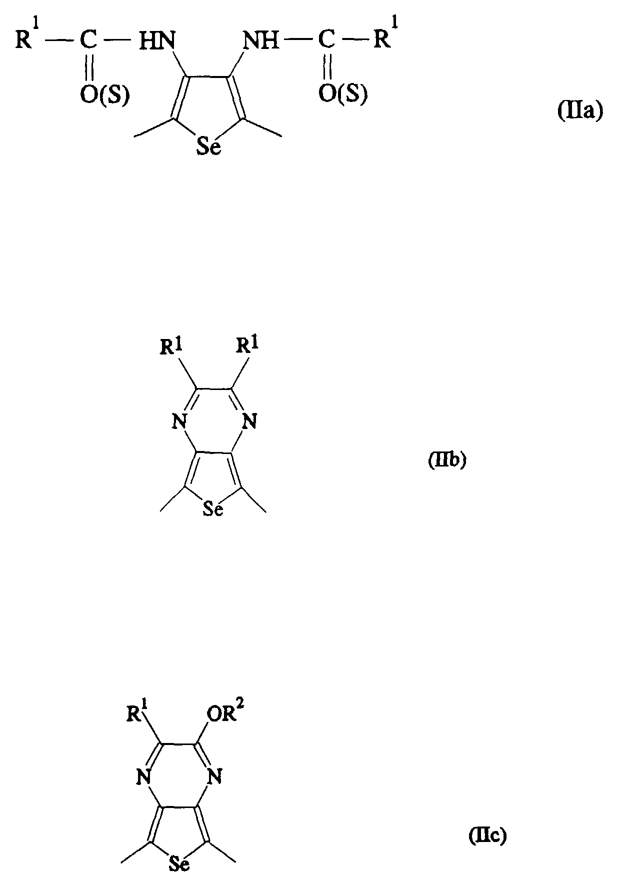 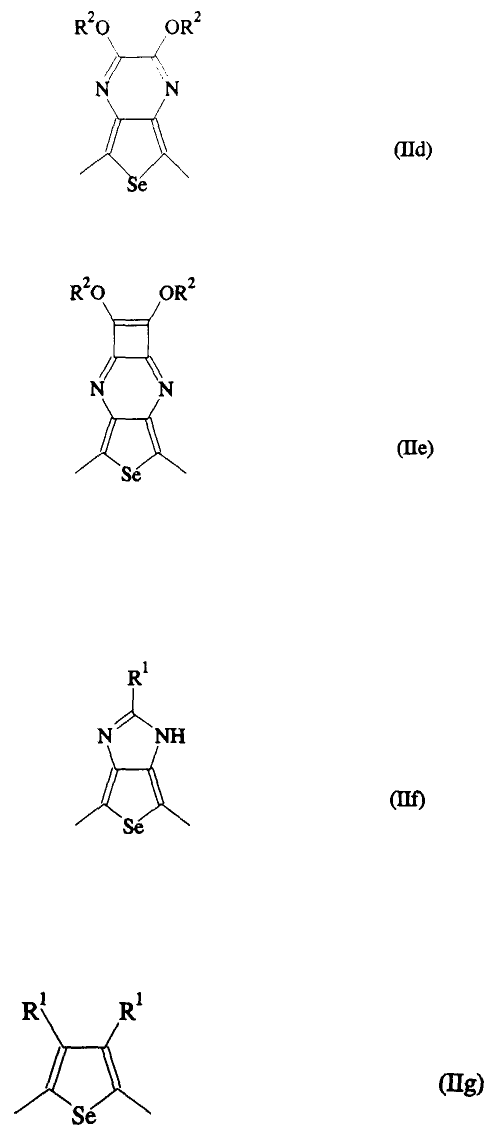 und Mischungen dieser, in denen R¹ wie in Anspruch 1 definiert ist und R² CHR¹₂ oder CH₂R¹ ist.

4. Verbindungen der allgemeinen Formeln (IXa) bis (IXc) 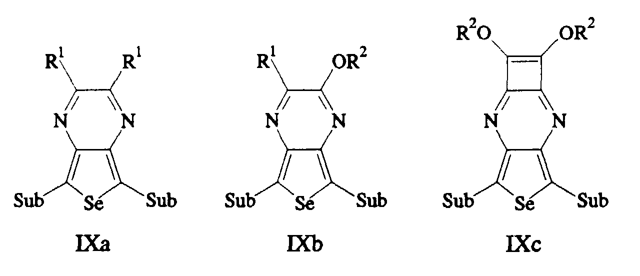 in der "Sub" Wasserstoff (H), Brom (Br), Chlor (Cl), Iod (I), Triflat (CF₃SO₃) oder Trialkylzinn ist, und R¹ wie in Anspruch 1 definiert ist und R² CHR¹₂ oder CH₂R¹ ist.

5. N,N-substituiertes 3,4-Diamino-2,5-disubstuituiertes Selenophen der allgemeinen Formel (X) 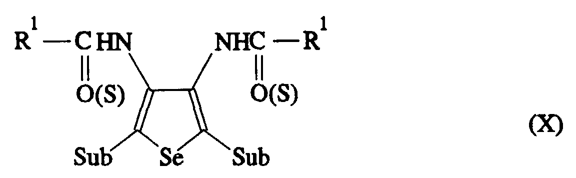 in der "Sub" wie in Anspruch 4 und R¹ wie in Anspruch 1 definiert ist.

6. 2,5-Bis(trialkylzinn)selenophen der allgemeinen Formel (XIa) 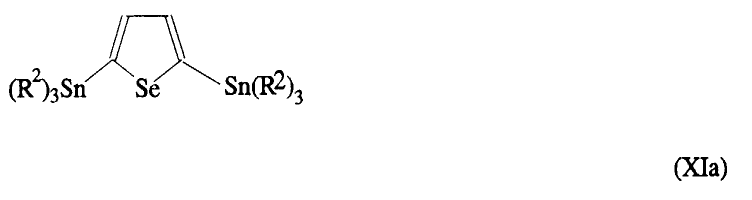 2,5'-Bis(trialkylzinn)-5,2'-diselenophen der allgemeinen Formel (XIb) 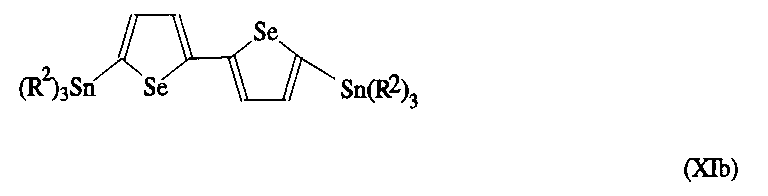 5,5''-Bis(trialkylzinn)-2,2':5',2''-Terselenophen der allgemeinen Formel (XIc) 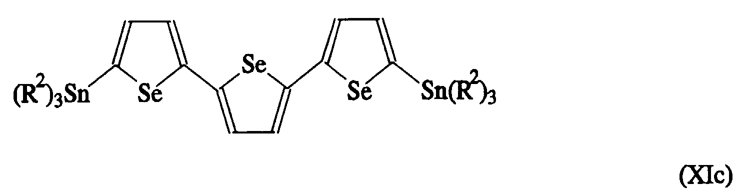 2-(Trialkylzinn)selenophen der allgemeinen Formel (XId) 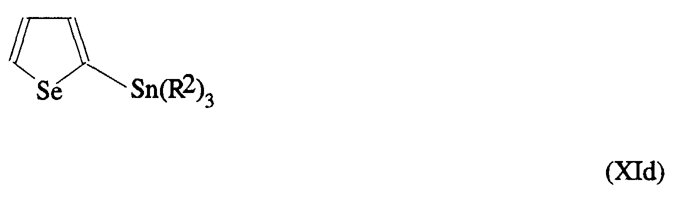
wobei R² jeweils eine Alkylgruppe darstellt,;
2,2'-Biselenophen, und 2,2':5',2''-Terselenophen.

7. Verfahren zur Herstellung eines Polyselenophens, wobei 2,5-Dihalogenselenophen oder 2,5-Ditriflatselenophen und an den dem Selen benachbarten Kohlenstoffatomen bis(Trialkylzinn)-substituierte Selenophenderivate, die der in Anspruch 1 definierten Struktureinheit (II) entsprechen, miteinander in geeigneten Lösungsmitteln in Anwesenheit von geeigneten Pd(0)- oder Pd(II)-Komplexen als Katalysator umgesetzt werden.

8. Verfahren zur Herstellung eines Polyselenophens, dadurch gekennzeichnet, daß 2,5-Bis(trialkylzinn)selenophen und an den dem Selen benachbarten Kohlenstoffatomen bis(Halogen)- oder bis(Triflat)-substituierte Selenophenderivate, die der in Anspruch 1 definierten Struktureinheit (II) entsprechen, miteinander in geeigneten Lösungsmitteln in Anwesenheit von geeigneten Pd(0)- oder Pd(II)-Komplexen als Katalysator umgesetzt werden.

9. Verwendung eines undotierten oder dotierten Polyselenophens gemäß einem der Ansprüche 1 bis 3 zur antistatischen Ausrüstung von den elektrischen Strom nicht oder nur schlecht leitenden Substanzen; als elektrisch leitfähige Folien; als Halbleiterfolien; als Zusatzstoff für aktive Elektroden; als LED's; als organische Transistoren; oder als Kondensatoren.

10. Verfahren zur antistatischen Ausrüstung eines den elektrischen Strom nicht oder nur schlecht leitenden Substrats durch Aufbringen einer Schicht enthaltend ein elektrisch leitfähiges organisches Polymer auf die Oberfläche des Substrats, wobei man auf der Oberfläche des Substrats durch Polymerisation eine Schicht mindestens eines Polyselenophens erzeugt, das eine oder mehrere Struktureinheiten der allgemeinen Formeln (I) oder (II) oder (I) und (II), wie in Anspruch 1 definiert, enthält.

11. Elektrisch leitfähiges Material, enthaltend mindestens ein Polyselenophen gemäß einem der Ansprüche 1 bis 3.
